# DEMANDE DE BREVET EUROPEEN

(11) **EP 0 898 946 A1**
(43) Date de publication de la demande: **03.03.1999**
(21) Numéro de dépôt: 98402141.0
(22) Date de dépôt: 28.08.1998
(51) Int. Cl.: A61F 2/40

(54) **Prothèse de l'extrémité supérieure de l'humérus**

(30) Priorité: 29.08.1997 FR 9710815
(71) Demandeur: Stratec Medical S.A., 75611 Paris Cedex 12 (FR)
(72) Inventeur: Alnot, Jean-Yves, 92100 Boulogne (FR); Augereau, Bernard, 92200 Neuilly Sur Deine (FR); Huten, Denis, 94300 Vincennes (FR); Hardy, Philippe, 75016 Paris (FR); Nordin, Yves, 75014 Paris (FR); Thomazeau, Hervé, 35000 Rennes (FR)
(74) Mandataire: Phélip, Bruno

(57) **Abrégé**

L'invention consiste en une prothèse modulaire de l'extrémité supérieure de l'humérus comprenant une tige médullaire (10) une tête humérale (30) et une entretoise métaphysaire (20) destinée à être fixée entre la tige et la tête, caractérisée en ce qu'elle comprend des moyens (24, 33) pour régler le déport antéro-postérieur de la tête humérale par rapport à la tige médullaire, indépendamment des autres paramètres de la prothèse.

## Description

L'invention concerne une prothèse de l'extrémité supérieure de l'humérus, du type comprenant une tige destinée à être ancrée dans le canal médullaire et une tête humérale destinée à coopérer avec la partie glénoïdienne de l'articulation.

Elle permet le traitement chirurgical des fractures articulaires de la tête humérale ou des ostéonécroses, des arthroses et des arthrites rhumatoïdes.

Cette prothèse peut également être mise en place sur un patient, en association avec une glène prothétique qui peut notamment être réalisée en polyéthylène.

Dans ce cas, elle permet le traitement chirurgical des douleurs induites par une incongruence de l'articulation gléno-humérale.

De nombreuses prothèses humérales sont déjà connues.

On peut citer les prothèses dites de Neer. Ces prothèses non contraintes, placées dans des conditions idéales, fonctionnent de manière identique à une épaule saine.

Cependant, ces prothèses sont du type monobloc, ce qui oblige à détenir en stock un nombre important d'implants pour que ceux-ci soient adaptés à chaque patient.

Par ailleurs, ces prothèses ne tiennent pas compte de toutes les particularités et variations anatomiques. Ainsi, les prothèses ne sont disponibles qu'en trois tailles de tige et deux tailles de tête humérale. Cette dernière est dans une position figée par rapport à la tige qui ne permet aucun déport.

L'inclinaison de la tête est également fixe et elle ne couvre pas de manière satisfaisante le plan de coupe de l'extrémité de l'humérus. Il est alors nécessaire d'adapter l'os à la prothèse, ce qui n'est pas souhaitable pour le patient.

Une autre prothèse est décrite dans le document FR-2 685 633.

Cette prothèse est modulaire et comprend trois éléments : une tige humérale d'ancrage, une tête humérale prenant la forme d'une calotte sphérique et une entretoise métaphysaire placée entre la tige et la calotte.

Elle peut aussi bien être appliquée à un bras droit qu'à un bras gauche.

Elle comprend également des moyens pour faire varier la position angulaire de la calotte humérale autour d'un axe géométrique excentré par rapport à son propre axe géométrique.

Cette variation angulaire conduit à modifier simultanément le déport antéro-postérieur et le déport latéral.

Ceci constitue la principale critique qui est formulée pour cette prothèse.

Enfin, chaque élément de la prothèse est de conception relativement complexe, ce qui induit des coûts importants.

L'invention est ainsi relative à une nouvelle prothèse modulaire de l'extrémité supérieure de l'humérus, de fabrication simple, comprenant des moyens pour régler le déport antéro-postérieur de la tête humérale par rapport à la tige, indépendamment des autres paramètres de la prothèse qui sont susceptibles d'être modifiés, et en particulier le déport latéral.

Les caractéristiques suivantes de l'invention peuvent également être prises en considération, isolément ou selon toute combinaison techniquement possible :
- les moyens pour régler le déport antéro-postérieur comprennent un tenon porté par l'entretoise métaphysaire et destiné à être fixé, dans une position variable, dans un évidement pratiqué dans la tête humérale et s'étendant dans un plan antéro-postérieur,
- la prothèse comprend une crémaillère sur la tête humérale et une autre crémaillère sur l'entretoise métaphysaire, s'étendant dans un plan antéro-postérieur et destinées à coopérer entre elles pour fixer le tenon dans une position déterminée dans l'évidement,
- la prothèse comprend des moyens pour immobiliser la tête humérale par rapport à l'entretoise métaphysaire,
- lesdits moyens d'immobilisation sont constitués par deux vis à bout conique qui sont destinées à s'engager dans un évidement de la tête humérale,
- la tige médullaire est de forme générale cylindrique et comporte, à une extrémité, des moyens de centrage sur l'entretoise métaphysaire,
- l'entretoise métaphysaire comporte un premier évidement débouchant sur une de ses faces extrêmes pour recevoir lesdits moyens de centrage,
- l'entretoise métaphysaire comprend un évidement qui est destiné à recevoir des moyens de fixation, lesquels pénètrent également dans un évidement de la tige médullaire,
- la tige médullaire comporte des rainures longitudinales,
- l'entretoise métaphysaire est délimitée par deux faces extrêmes qui forment entre elles un angle α compris entre 120 et 140°,
- la tête humérale est délimitée par une base et une face extérieure dont la courbure dans le plan sagittal est différente de celle dans le plan frontal.

L'invention sera mieux comprise et d'autres buts, avantages et caractéristiques de celle-ci apparaîtront plus clairement à la lecture de la description suivante d'exemples non limitatifs de réalisation de l'invention et qui est faite au regard des dessins annexés sur lesquels :
- la figure 1 est une vue éclatée d'une prothèse conforme à l'invention,
- la figure 2 est une vue en coupe partielle d'une prothèse selon l'invention à l'état monté,
- la figure 3 est une vue de dessus de la tête humérale selon l'invention ; elle comporte les figures 3a, 3b et 3c qui illustrent trois déports antéro-postérieur différents,
- la figure 4 est une vue en coupe d'un humérus avec la prothèse selon l'invention mise en place.

Les éléments communs aux différentes figures seront désignés par les mêmes références.

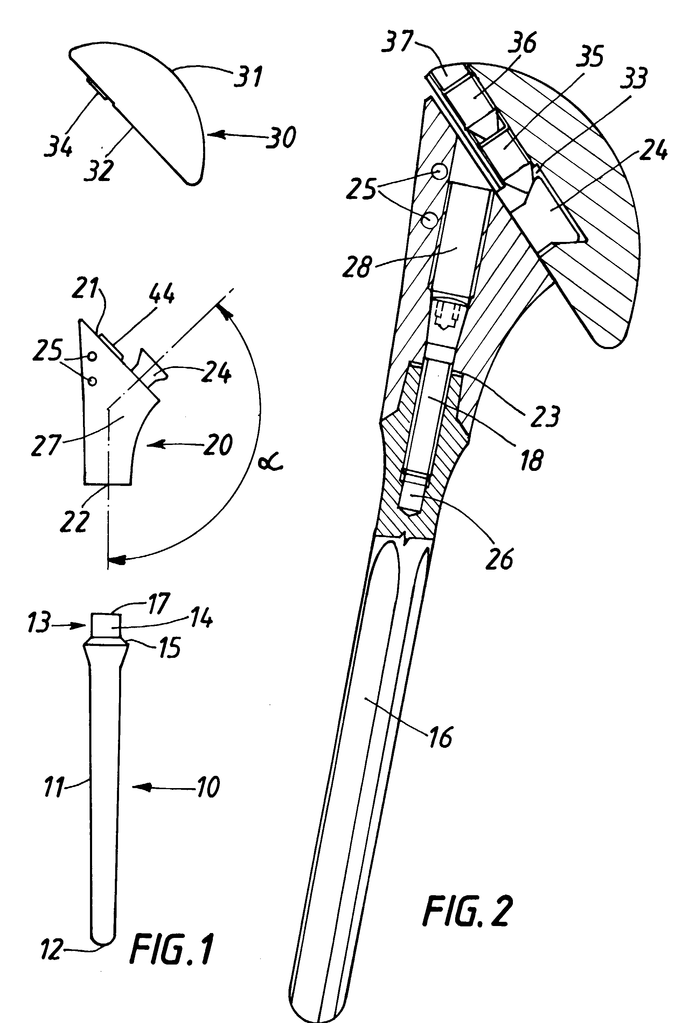

En référence à la figure 1, la prothèse modulaire selon l'invention est composée de trois éléments : une tige médullaire 10, une entretoise métaphysaire 20 et une tête humérale 30.

La tige médullaire 10 comporte un corps 11 présentant la forme générale d'un cylindre qui se termine d'un côté, par une extrémité arrondie 12 et de l'autre côté, par des moyens de centrage 13 destinés à être assemblés avec la métaphyse prothétique 20.

Dans l'exemple illustré sur les figures, la section du corps 11 s'élargit à proximité des moyens de centrage 13. La tige 10 présente donc une légère conicité.

Les moyens 13 sont par exemple constitués par une partie conique d'assemblage 14, raccordée au corps 11 au moyen d'une surface 15 inclinée.

Comme le montre la figure 2, le corps 11 de la tige médullaire comporte des rainures longitudinales 16, de préférence au nombre de quatre.

La tige médullaire 10 est destinée à être cimentée dans le canal médullaire de l'humérus opéré.

Les rainures 16 empêchent la rotation de la tige dans le canal médullaire, après son implantation. Par ailleurs, la légère conicité de la tige 10 facilite son extraction en cas de reprise.

La tige médullaire peut s'adapter indifféremment du côté droit ou du côté gauche d'un patient. Il est cependant souhaitable qu'elle soit disponible en plusieurs longueurs et/ou diamètres différents, pour s'adapter à l'anatomie du patient. La longueur de la tige peut notamment être comprise entre 120 et 155 cm, tandis que son diamètre sera par exemple compris entre 8 et 12 mm.

L'entretoise métaphysaire ou métaphyse prothétique 20 présente deux faces extrêmes 21 et 22 qui ne sont pas parallèles entre elles. On désigne par α l'angle entre les deux plans perpendiculaires aux faces extrêmes 21 et 22.

Dans l'exemple représenté à la figure 1, l'angle α est de 130°. Il peut être compris entre 120° et 140°. De préférence, la métaphyse prothétique peut présenter trois angles différents : 125°, 130° et 135°. Ces angles correspondent à différents cols anatomiques et sont choisis en fonction du patient opéré.

La première face extrême 22 est destinée à s'assembler avec les moyens de centrage 13 de la tige 10. A cet effet, la métaphyse prothétique 20 comporte un premier évidement conique 23 qui est sensiblement la forme en creux des moyens 13 et qui débouche sur la face 22.

Ceci permet aux moyens de centrage 13 de venir s'engager dans l'évidement 23, comme l'illustre la figure 2.

La deuxième face extrême 21 de la métaphyse prothétique est une face plane sur laquelle existe un tenon excentré 24. Il permet l'emboîtement de la métaphyse prothétique 20 sur la tête humérale 30.

Une crémaillère 44 est également prévue sur la deuxième face extrême 21, s'étendant dans un plan antéro-postérieur.

Dans l'exemple illustré sur les figures, elle comprend 6 dents d'un pas de 1 mm, avec une hauteur de dent de 0,8 mm et une largeur de 8 mm. Elle contribue à positionner la tête humérale 30 sur la métaphyse 20.

La métaphyse 20 présente une paroi postérieure (non illustrée sur les figures) et une paroi antérieure 27. Elle est anatomique et une métaphyse différente doit être utilisée respectivement pour le côté droit ou le côté gauche d'un patient.

Par ailleurs, la métaphyse prothétique comporte deux trous latéraux 25 qui joignent la paroi antérieure 27 à la paroi postérieure.

Ils permettent de fixer les fragments osseux (trochin et trochiter) en cas de fracture complexe.

Comme l'illustre la figure 2, l'entretoise métaphysaire 20 comporte un deuxième évidement 28 qui débouche d'un côté, dans le premier évidement 23 et de l'autre côté, sur la deuxième face extrême 21.

Ce deuxième évidement 28 présente une forme générale cylindrique.

La tige médullaire 10 comporte également un évidement 26 qui débouche sur la face externe 17 du cône d'assemblage 14. Cet évidement présente une forme générale cylindrique. Il s'étend sur quelques centimètres. Dans l'exemple illustré à la figure 2, l'évidement 26 est centré sur l'axe de symétrie de la tige médullaire 10.

Les évidements 28 et 26 prévus respectivement, dans la métaphyse prothétique 20 et dans la tige médullaire 10, sont conçus pour se trouver dans le prolongement l'un de l'autre, lorsque la métaphyse et la tige sont assemblées. C'est ce qu'illustre la figure 2.

Ainsi, une fois que les moyens de centrage 13 sont introduits dans l'évidement 23 de la métaphyse prothétique 20, des moyens de fixation 18 peuvent être introduits dans les évidements 28 et 26 pour rendre solidaires la tige humérale et la métaphyse prothétique.

Ces moyens de fixation peuvent notamment être constitués par une vis à tête conique 18.

La tête humérale 30 est délimitée par une face extérieure 31 et une base 32.

Elle n'est pas sphérique. La courbure de la face extérieure 31 dans le plan frontal est différente de celle dans le plan sagittal.

Elle permet ainsi de respecter au mieux l'anatomie du patient qui subit l'intervention. De préférence, plusieurs tailles sont disponibles à cet effet.

Comme le montre la figure 2, la tête humérale 30 comporte un évidement 33 qui débouche sur la base 32 de la tête.

Cet évidement 33 est conçu pour recevoir le tenon 24 de la métaphyse prothétique 20. Il s'étend dans un plan antéro-postérieur.

La tête humérale comporte également une crémaillère 34 prévue sur la base 32 et située environ au 1/3 supérieur de la tête. Dans un exemple de réalisation, elle comprend 27 dents d'un pas de 1 mm avec une hauteur de dent de 0,8 mm, et une largeur de 8 mm. Elle est centrée et s'étend dans un plan antéro-postérieur. Ainsi, les dents de la crémaillère 34 sont situées dans les plans latéraux. Elle permet, en coopération avec la crémaillère 44 prévue sur la métaphyse prothétique 20, de positionner la tête humérale 30 par rapport à la pièce métaphysaire 20, après avoir réglé le déport antéro-postérieur grâce au tenon 24.

Dans l'exemple illustré sur la figure 2, les moyens pour assurer la fixation de la tête humérale sur la métaphyse prothétique, sont constitués par deux vis à bout conique 35 et 36 qui sont engagées dans un évidement 37 pratiqué dans la tête humérale 30.

Cet évidement 37 est situé dans un plan sensiblement parallèle à la base 32 de la tête.

Le bout conique de la première vis 35 vient en contact avec le tenon 24 pour immobiliser la tête sur la métaphyse et la deuxième vis à bout conique 36 vient en contact avec la tête 35 pour la bloquer, se comportant en contre écrou.

D'autres moyens de fixation pourraient cependant être prévus.

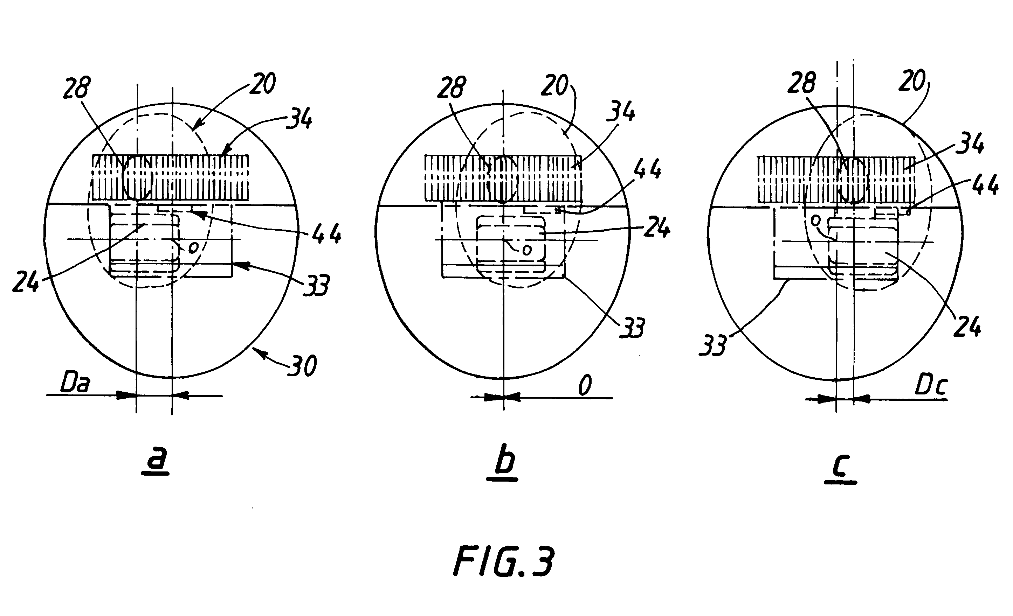

En référence à la figure 3, on va maintenant décrire des exemples de variation du déport, dans un plan antéro-postérieur, de la tête par rapport à la métaphyse humérale 20, en utilisant les moyens prévus sur la prothèse selon l'invention.

La figure 3 est une vue de dessus de la prothèse droite selon l'invention, sur laquelle la position de la pièce métaphysaire 20 est indiquée en traits pointillés et les formes cachées de la tête 30 en traits mixtes forts.

Chaque figure 3a et 3c illustre la position de la métaphyse droite dans chaque position extrême de déport antérieur (figure 3a) et de déport postérieur (figure 3c), étant entendu que la métaphyse 20 peut rendre toutes les positions intermédiaires entre ces deux positions extrêmes suivant le pas des crémaillères 44 et 34.

Dans la position illustrée à la figure 3a, l'axe longitudinal de la métaphyse 20 qui passe par le trou 28 est décalé antérieurement, d'une distance Da (environ 6mm) par rapport au centre 0 de la tête 30.

Dans la position illustrée à la figure 3b, l'axe longitudinal de la métaphyse 20 et le centre 0 de la tête sont dans un même plan latéro-médial.

Dans la position illustrée à la figure 3c, l'axe longitudinal de la métaphyse 20 qui passe par le trou 28 est décalée postérieurement, d'une distance Dc (environ 3mm) par rapport au centre 0 de la tête 30.

Le déport antéro-postérieur est ajusté en faisant varier la position du tenon 24 dans l'évidement 33.

En pratique, le déport varie de préférence en nombre entier de 1 mm correspondant au pas des crémaillères 44 et 34.
L'engagement des crémaillères permet de fixer le déport antéro-postérieur. L'immobilisation complète de la tête par rapport à la métaphyse prothétique 20 est ensuite obtenue grâce aux vis 35 et 36.

Dans l'exemple illustré à la figure 3, le déport maximal est de 6 mm en antérieur et 3 mm en postérieur, de part et d'autre d'une position moyenne.

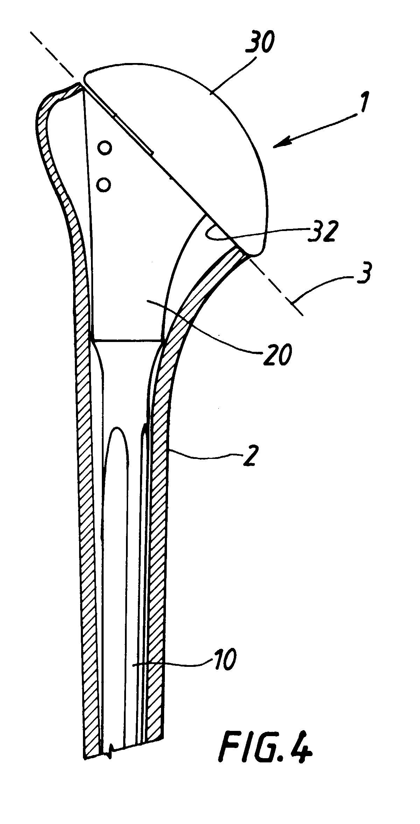

La figure 4 montre la prothèse 1 selon l'invention en place sur un humérus 2. On constate que la base 32 de la tête humérale 30 vient reposer sur le plan de coupe osseuse 3 qui est représenté en traits pointillés.

La conception modulaire de la prothèse permet de choisir de façon appropriée chaque élément qui la compose, de façon à tenir compte de l'anatomie du patient opéré.

Le chirurgien peut par ailleurs ajuster le déport antéro-postérieur de la tête de la prothèse, en fonction de l'anatomie du patient, sans que les autres paramètres soient modifiés, en particulier le déport latéral.

Chaque élément constitutif de la prothèse et les moyens pour régler le déport sont de construction simple, ce qui rend la prothèse selon l'invention facile à fabriquer et donc de coût réduit.

Les signes de référence insérés après les caractéristiques techniques mentionnées dans les revendications ont pour seul but de faciliter la compréhension de ces dernières et ne sauraient en limiter la portée.

## Revendications

1. Prothèse modulaire de l'extrémité supérieure de l'humérus comprenant une tige médullaire (10), une tête humérale (30) et une entretoise métaphysaire (20) destinée à être fixée entre la tige et la tête, caractérisée en ce qu'elle comprend des moyens (24, 33) pour régler le déport antéro-postérieur de la tête humérale par rapport à la tige médullaire, indépendamment des autres paramètres de la prothèse.

2. Prothèse modulaire selon la revendication 1, caractérisée en ce que les moyens pour régler le déport antéro-postérieur comprennent un tenon (24) porté par l'entretoise métaphysaire (20) et destiné à être fixé, dans une position variable, dans un évidement (33) pratiqué dans la tête humérale (30) et s'étendant dans un plan antéro-postérieur.

3. Prothèse modulaire selon la revendication 2, caractérisée en ce qu'elle comprend une crémaillère (34) sur la tête humérale (30) et une autre crémaillère (44) sur l'entretoise métaphysaire (20), s'étendant dans un plan antéro-postérieur et destinées à coopérer entre elles pour fixer le tenon (24) dans une position déterminée dans l'évidement (33).

4. Prothèse modulaire selon l'une des revendications 1 à 3, caractérisée en ce qu'elle comprend des moyens (35, 36, 37) pour immobiliser la tête humérale (30) par rapport à l'entretoise métaphysaire (20).

5. Prothèse modulaire selon la revendication 4, caractérisée en ce que lesdits moyens d'immobilisation sont constitués par deux vis à bout conique (35, 36) qui sont destinées à s'engager dans un évidement (37) de la tête humérale (30).

6. Prothèse modulaire selon l'une des revendications 1 à 5, caractérisé en ce que la tige médullaire (10) est de forme générale cylindrique et comporte, à une extrémité, des moyens de centrage (13) sur l'entretoise métaphysaire (20).

7. Prothèse modulaire selon la revendication 6, caractérisée en ce que l'entretoise métaphysaire (20) comporte un premier évidement (23) débouchant sur une de ses faces extrêmes (22) pour recevoir lesdits moyens de centrage (13).

8. Prothèse modulaire selon l'une des revendications 1 à 7, caractérisée en ce que l'entretoise métaphysaire (20) comprend un évidement (28) qui est destiné à recevoir des moyens de fixation (18), lesquels pénètrent également dans un évidement (26) de la tige médullaire (10).

9. Prothèse modulaire selon l'une des revendications 1 à 8, caractérisée en ce que la tige médullaire (10) comporte des rainures longitudinales (16).

10. Prothèse modulaire selon l'une des revendications 1 à 9, caractérisée en ce que l'entretoise métaphysaire (20) est délimitée par deux faces extrêmes (21, 22) qui forment entre elles un angle α compris entre 120 et 140°.

11. Prothèse modulaire selon l'une des revendications 1 à 10, caractérisée en ce que la tête humérale (30) est délimitée par une base (32) et une face extérieure (31) dont la courbure dans le plan sagittal est différente de celle dans le plan frontal.
